# EUROPEAN PATENT SPECIFICATION

(11) **EP 4 215 246 B1**
(45) Date of publication and mention of the grant of the patent: **16.09.2026**
(21) Application number: 23152345.7
(22) Date of filing: 19.01.2023
(51) Int. Cl.: A61Q 19/10, A61Q 1/14, A61Q 5/02, A61K 8/73, A61K 8/41, A61K 8/44, A61K 8/42, A61K 8/04

(54) **PERSONAL CARE COMPOSITION WITH LOW SUBSTITUTED HYDROXYPROPYL CELLULOSE**
KÖRPERPFLEGEZUSAMMENSETZUNG MIT GERING SUBSTITUIERTER HYDROXYPROPYLCELLULOSE
COMPOSITION DE SOINS PERSONNELS AVEC DE L'HYDROXYPROPYLCELLULOSE FAIBLEMENT SUBSTITUÉE

(30) Priority: 19.01.2022 JP 2022006101
(43) Date of publication of application: 26.07.2023
(73) Proprietor: SHIN-ETSU CHEMICAL CO., LTD., Tokyo 100-0005 (JP)
(72) Inventor: SUDO, Takane, Joetsu-shi, Niigata, 9428601 (JP); HIRAMA, Yasuyuki, Joetsu-shi, Niigata, 942860 (JP); NINOBE, Shingo, Joetsu-shi, Niigata, 942860 (JP)
(74) Representative: Schulz Junghans Patentanwälte PartGmbB

(56) References cited:
- JP-A- 2006 151 992
- US-A1- 2003 166 918
- KLEINEBUDDE ET AL: "Application of low substituted hydroxypropylcellulose (L-HPC) in the production of pellets using extrusion/spheronization", INTERNATIONAL JOURNAL OF PHARMACEUTICS, ELSEVIER, AMSTERDAM, NL, vol. 96, no. 1-3, 31 July 1993 (1993-07-31), pages 119 - 128, XP025565603, ISSN: 0378-5173, [retrieved on 19930731], DOI: 10.1016/0378-5173(93)90219-6

## Description

### Technical Field

The present invention relates to a personal care composition and its use in a product provided to cleanse a subject's body.

### Background Art

Personal care compositions containing scrubbing agents have been known to be used for the purposes including removing dirt and oil on the skin, improving the appearance of the skin and providing a moderate massage effect.

Known scrubbing agents employed in the personal care compositions include synthetic resins such as polystyrene, polyethylene and nylon (e.g., Patent Document 1), crushed products of plant seeds or seed husks such as apricot, peach and walnut (e.g., Patent Document 2), and water-absorbent swellable polymers such as glucomannan (e.g., Patent Document 3). See also US 2003/166918 A1 (OBARA SAKAE [JP]) 4 September 2003 (2003-09-04).

### Citation List

### Patent Documents

Patent Document 1: JP 2016-017048 A
Patent Document 2: JP H10-338625 A
Patent Document 3: JP 2000-344801 A

### Summary of the Invention

### Problems to be Solved by the Invention

However, since the synthetic resins such as polystyrene employed in Patent Document 1 are often persistent, the personal care compositions containing such synthetic resins cause marine pollution and have a high environmental impact.

The crushed products of plant seeds and seed husks, such as apricot hulls, employed in Patent Document 2 are relatively hard. Therefore, the personal care compositions containing such crushed products have an excellent cleansing property, but have a poor skin feel.

The personal care compositions containing water-absorbent swellable particles such as glucomannan employed in Patent Document 3 have a good skin feel, but have a poor cleansing property.

In view of the above circumstances, it is an object of the present invention to provide a personal care composition having a low environmental impact, a good skin feel and a high cleansing property.

### Means of Solving the Problems

In order to solve the above-identified problems, the present inventors earnestly examined to find an appropriate scrubbing agent, although thousands or tens of thousands of scrubbing agents are said to exist. The present inventors then turned their attention to low substituted hydroxypropyl cellulose as a material that is both biodegradable and swellable. However, many of personal care compositions containing low substituted hydroxypropyl cellulose tended to have a relatively good skin feel but to show a poor cleansing property.

As a result of the further trial and error process, the present inventors surprisingly found that the personal care compositions containing low-substituted hydroxypropyl cellulose having a particle size and an (average) aspect ratio, which are within a certain range in the dry state, respectively, not only have a good skin feel but also show an excellent cleansing property.

More surprisingly, the present inventors found that by rendering the content of hydroxypropoxy group contained in low substituted hydroxypropyl cellulose within a certain range, the resulting personal care compositions exhibited a more excellent cleansing property.

Finally, based on such findings, the present inventors have succeeded in inventing a personal care composition containing low substituted hydroxypropyl cellulose and water, wherein the low substituted hydroxypropyl cellulose has a particle size within a certain range and an aspect ratio within a certain range in the dry state, or the low substituted hydroxypropyl cellulose has a particle size within a certain range and an aspect ratio within a certain range in the dry state as well as has the content of hydroxypropoxy group within a certain range. As such, the present invention has been completed on the basis of the findings and successful examples that were first found or obtained by the present inventors.

According to the present invention, there are provided personal care compositions and methods as defined in the appended claims.

### Effects of the Invention

According to the present invention, it can be achieved to provide a personal care composition having not only a low environmental impact and a good skin feel but also a high cleansing property. The personal care composition or method according to one embodiment of the present invention can be applied on a daily base to the skin so that the condition of the skin can be maintained or improved to a better state.

### Description of Embodiments

While each embodiment of the present invention will now be described in detail, the present invention is defined by the appended claims.

Unless otherwise specified, each term used herein is used in the meaning commonly used by those skilled in the art, in particular in the cellulose, toiletry and cosmetics fields, and should not be construed to have any meaning that is unduly limiting. Also, any speculations and theories herein are made on the basis of the knowledge and experiences of the present inventors and as such, the present invention is not bound by any such speculations and theories.

While the term "composition" is not particularly limited and means composition as well known, it is, for example, comprised of combination of two or more components.

The term "personal care" means the care of a body, and the term "personal care composition" means a composition used for caring a body.

The term "and/or" as used herein means either any one of, any combination of two or more of, or combination of all of listed related items.

The term "content" is synonymous with concentration and amount used (amount added), and means the ratio of the amount of a component relative to the total amount of the composition. Unless otherwise specified, the unit of content used herein means "% by mass", "wt%" and "%(w/w)". However, the total content of the components may not exceed 100 %.

The wording "to" for indicating a range of values is intended to include values preceding and following the wording; for example, "0 % to 100 %" means a range from 0 % or more and 100 % or less. The terms "more than" and "less than" used herein means the lower and upper limits without including a value following the term, respectively. For example, "more than 1" means a value beyond 1, and "less than 100" means a value below 100.

The terms "include," "comprise," and "contain" mean that an element(s) other than an element(s) as explicitly indicated can be added as inclusions, which are, for example, synonymous with "at least include," but encompasses the meaning of "consist of" and "substantially consist of". In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

The number of digits of an integer equals to its significant figure. For example, 1 has one significant figure and 10 has two significant figures. For a decimal number, the number of digits after a decimal point equals to its significant figure. For example, 0.1 has one significant figure and 0.10 has two significant figures.

### <Summary of personal care composition>

The personal care composition according to one embodiment of the present invention contains water and low substituted hydroxypropyl cellulose. The personal care composition according to one embodiment of the present invention employs as a scrubbing agent low substituted hydroxypropyl cellulose that is biodegradable and swellable, and has a cleansing property. As a result, the personal care composition can be a personal care composition having not only a low environmental impact and a good skin feel but also an excellent cleansing property.

### <Low substituted hydroxypropyl cellulose>

In the personal care composition according to one embodiment of the present invention, low-substituted hydroxypropyl cellulose, which is not soluble in water but has the property of absorbing water and swelling thereby, is employed as a scrubbing agent.

In general, cellulose is insoluble in water. However, cellulose derivatives with alkoxy groups and/or hydroxyalkoxy groups can have water solubility depending on the degree of substitution. Such cellulose derivatives can be obtained by replacing the hydrogen atom of the hydroxyl group of the glucose ring constituting cellulose with the alkyl group or hydroxyalkyl group. In addition, cellulose derivatives with the lower degree of substitution do not show water solubility and may have the property of absorbing water and swelling thereby (swellability). Low substituted hydroxypropyl cellulose, which is a type of cellulose derivatives, tends to have swellability because of its lower degree of substitution of hydroxypropoxy group.

The pulverized low substituted hydroxypropyl cellulose with swellability become partially swollen when dispersed in water. In contrast, cellulose derivatives with the higher degree of substitution of hydroxypropoxy group become soluble in water. Even if such water-soluble hydroxypropyl cellulose is employed, it is not possible to obtain a personal care composition that has the property of removing dirt and oil from the skin and the other body (cleansing property). Therefore, low substituted hydroxypropyl cellulose employed in the personal care composition according to one embodiment of the present invention in anticipation of a scrubbing action is a low substituted hydroxypropyl cellulose with swellability.

The personal care composition according to one embodiment of the present invention is characterized by containing such a low substituted hydroxypropyl cellulose that not only has swellability but also can impart an excellent cleansing property to the personal care composition.

Since the low substituted hydroxypropyl cellulose is used to impart an excellent cleansing property to the personal care composition, such a low substituted hydroxypropyl cellulose has an average particle size (D₅₀) on a volume basis by dry laser diffraction of 30 µm to 200 µm, preferably 35 µm to 150 µm, more preferably 35 µm to 100 µm, still more preferably 35 µm to 60 µm, and even still more preferably 35 µm to 55 µm.

A personal care composition containing low substituted hydroxypropyl cellulose with an average particle size (D₅₀) on a volume basis by dry laser diffraction of less than 30 µm may demonstrate no sufficient cleansing effect due to the reason that the low substituted hydroxypropyl cellulose is smaller than dirt. A personal care composition containing low substituted hydroxypropyl cellulose with an average particle size (D₅₀) on a volume basis by dry laser diffraction of more than 200 µm, may cause a strongly irritating sensation to the skin and demonstrate no sufficient cleansing effect due to the reason that the low substituted hydroxypropyl cellulose has difficulty in getting into pores in the skin.

The average particle size (D₅₀) on a volume basis by dry laser diffraction of low substituted hydroxypropyl cellulose is measured according to the method in the section "D₅₀ by dry method" as described in Examples below.

Since the low substituted hydroxypropyl cellulose is used to impart an excellent cleansing property to the personal care composition, the low substituted hydroxypropyl cellulose has an (average; see measurement in Examples) aspect ratio equal to or more than 1.0 and less than 5.0, preferably 1.0 to 4.5, more preferably 1.0 to 4.0, still more preferably 1.5 to 4.0, and even still more preferably 2.0 to 4.0.

If the personal care composition contains low substituted hydroxypropyl cellulose having an (average) aspect ratio equal to or more than 5.0, it may cause a strongly irritating sensation to the skin, and further demonstrate no sufficient cleansing effect because the low substituted hydroxypropyl cellulose has difficulty in getting into pores in the skin.

The aspect ratio of low substituted hydroxypropyl cellulose refers to the ratio (L/D) of the long diameter (L) relative to the short diameter (D) measured from scanning electron micrographs. The aspect ratio of low substituted hydroxypropyl cellulose is measured according to the method in the section "Aspect ratio" as described in Examples below.

The low substituted hydroxypropyl cellulose may have advantageous properties as a component that provides a scrubbing action in the personal care composition.

For example, the section "Low substituted hydroxypropyl cellulose" in Japanese Pharmacopoeia, 18th Edition describes that the content of hydroxypropoxy group in low substituted hydroxypropyl cellulose is in the range between 4.0 % by mass and 16.0 % by mass. However, the present inventors found the tendency that a lower content of hydroxypropoxy groups of the low substituted hydroxypropyl cellulose employed correlated to a more excellent cleansing property of the resulting personal care composition. Therefore, the low substituted hydroxypropyl cellulose may have, for example, the content of hydroxypropoxy group of preferably 4.0 % by mass to 11.0 % by mass, more preferably from 4.5 % by mass to 11.0 % by mass, and still more preferably 5.0 % by mass to 11.0 % by mass.

In certain particular embodiments, the low substituted hydroxypropyl cellulose has a content of hydroxypropoxy group of 4.5 % by mass to 10.5 % by mass. In certain more particular embodiments, the low substituted hydroxypropyl cellulose has a content of hydroxypropoxy group of 5.0 % by mass to 9.5 % by mass. These values may be combined with any values of average particle size (D₅₀) in dry or swollen form disclosed herein, and also with any values for average aspect ratio of the particles in dry or swollen form, as well as with any mass ratio value given herein.

The content of hydroxypropoxy group of less than 4.0 % by mass may render the low substituted hydroxypropyl cellulose less swellable. As a result, the obtained personal care composition may have the problems that the skin feel becomes deteriorated and the low substituted hydroxypropyl cellulose becomes settled out in the personal care composition. To the contrary, the content of hydroxypropoxy group of more than 11.0 % by mass may render the low substituted hydroxypropyl cellulose more swellable. As a result, the obtained personal care composition may demonstrate no good cleansing property due to having too soft particles of the scrubbing agent.

The content of hydroxypropoxy group in low substituted hydroxypropyl cellulose is measured according to the measurement method for low substituted hydroxypropyl cellulose in Japanese Pharmacopoeia, 18th Edition, as described in the section "Assay" (Content of hydroxypropoxy group) in Examples below.

The low substituted hydroxypropyl cellulose that is in the swollen state in the personal care composition may have an average particle size (D₅₀) on a volume basis by wet laser diffraction of preferably 50 µm to 400 µm, more preferably 55 µm to 300 µm, still more preferably 60 µm to 200 µm, and even still more preferably 60 µm to 150 µm, 60 µm to 120 µm or 60 µm to 100 µm from the viewpoint of the cleansing property and skin feel provided to the personal care composition.

The swollen state of the low substituted hydroxypropyl cellulose contained in the personal care composition means the state that the particle of low substituted hydroxypropyl cellulose has absorbed water and water has penetrated into the particles so that the particle size is larger than that in the dry state.

One specific embodiment of low substituted hydroxypropyl cellulose has an average particle size (D₅₀) on a volume basis by dry laser diffraction of 30 µm to 200 µm and an aspect ratio equal to or more than 1.0 and less than 5.0; or in addition to these properties, has the content of hydroxypropoxy group of 4.0 % by mass to 11.0 % by mass; and/or when being in the swollen state in the personal care composition, has an average particle size (D₅₀) on a volume basis by wet laser diffraction of 50 µm to 400 µm.

Another specific embodiment of low substituted hydroxypropyl cellulose has an average particle size (D₅₀) on a volume basis by dry laser diffraction of 35 µm to 100 µm and an aspect ratio of 1.0 to 4.0; or in addition to these properties, has the content of hydroxypropoxy group of 4.5 % by mass to 11.0 % by mass; and/or when being in the swollen state in the personal care composition, has an average particle size (D₅₀) on a volume basis by wet laser diffraction of 60 µm to 150 µm.

Another specific embodiment of low substituted hydroxypropyl cellulose has an average particle size (D₅₀) on a volume basis by dry laser diffraction of 35 µm to 55 µm and an aspect ratio of 2.0 to 4.0; or in addition to these properties, has the content of hydroxypropoxy group of 5.0 % by mass to 11.0 % by mass; and/or when being in the swollen state in the personal care composition, has an average particle size (D₅₀) on a volume basis by wet laser diffraction of 60 µm to 100 µm.

The method of producing low substituted hydroxypropyl cellulose may be any known method but be not particularly limited. Examples of the method of producing low substituted hydroxypropyl cellulose include the method described in JP S57-53100 B. One non-limited specific example of the method of producing low substituted hydroxypropyl cellulose is shown below.

First, alkali cellulose is prepared. Alkali cellulose is prepared by soaking the starting material sheet pulp in an aqueous alkali solution such as caustic soda, by adding the pulverized pulp to the aqueous alkali solution and mixing them, or by dispersing the pulverized pulp in an organic solvent and then adding an alkali to the resulting dispersion.

The alkali cellulose and a hydroxypropylating agent such as propylene oxide are then added to a reactor, and the reactor is heated to react the components to obtain a cellulose ether reaction product.

The resulting cellulose ether reaction product is transferred to another tank, and the alkali in the tank is neutralized with an acid to obtain a solid low substituted hydroxypropyl cellulose. The solid product is then washed, dried and pulverized to obtain low substituted hydroxypropyl cellulose in the powder form. Alternatively, the cellulose ether reaction product after the reaction is added to water or a mixed solution composed of water and an acid so that the cellulose ether reaction product can be completely or partially dissolved. The mixture is neutralized by adding an acid thereto to precipitate a crude low substituted hydroxypropyl cellulose. The precipitated crude low substituted hydroxypropyl cellulose is washed, dried and pulverized to obtain low substituted hydroxypropyl cellulose in the powder form.

While the content of low substituted hydroxypropyl cellulose is not particularly limited, examples of the content include, relative to the total mass of the personal care composition, preferably 0.01 % by mass to 30.00 % by mass, more preferably 0.10 % by mass to 20.00 % by mass, and still more preferably 1.00 % by mass to 10.00 % by mass, from the viewpoint of the cleansing property and viscosity provided to the personal care composition.
In certain embodiments, the content of low substituted hydroxypropyl cellulose, relative to the total mass of the personal care composition, is in the range of 3.0 % to 8.0 % by mass. In certain particular embodiments, the content of low substituted hydroxypropyl cellulose, relative to the total mass of the personal care composition, is in the range of 4.0 % to 6.0 % by mass.

As long as the problem of the present invention can be solved, the personal care composition may contain, in addition to the above-mentioned low substituted hydroxypropyl cellulose, another low substituted hydroxypropyl cellulose with other properties different from those of the low substituted hydroxypropyl cellulose, for example, the low substituted hydroxypropyl cellulose having an average particle size (D₅₀) on a volume basis by dry laser diffraction of less than 30 µm or more than 200 µm and/or having an aspect ratio of less than 1.0 or equal to or more than 5.0. In addition, it is preferred that the low substituted hydroxypropyl cellulose in the personal care composition consists of or substantially consists of the above-mentioned low substituted hydroxypropyl cellulose.

### <Water>

Water is not particularly limited as long as water is one normally used in the process of manufacturing a personal care composition. Examples of water include tap water and purified water, preferably purified water because of not containing impurities.

The content of water is not particularly limited as long as the content is one normally used in a personal care composition.

For example, in order that the low substituted hydroxypropyl cellulose can be sufficiently swollen in the personal care composition, the ratio of the mass of water relative to the mass of low substituted hydroxypropyl cellulose is preferably equal to or more than 3.0, more preferably 4.5 to 99.0, still more preferably 6.0 to 95.0, and even still more preferably 7.5 to 90.0.

Examples of the content of water in the personal care composition include, from the viewpoint of the swellability of low substituted hydroxypropyl cellulose as well as the skin irritation, cleansing property and stability of the personal care composition, preferably 2.0 % by mass to 99.0 % by mass, more preferably 3.0 % by mass to 95.0 % by mass, and still more preferably 5.0 % by mass to 90.0 % by mass. Since the low substituted hydroxypropyl cellulose in the personal care composition does not need to be in the completely swollen state, the content of water may be about twice the amount of low substituted hydroxypropyl cellulose.

### <Surfactant>

It is preferred that the personal care composition according to one embodiment of the present invention further contains a surfactant in order to enhance the cleansing property.

The surfactant is not particularly limited, and may be any type of surfactants normally employed as a component in a personal care composition. Examples of the surfactant include anionic surfactants, cationic surfactants, ampholytic surfactants and nonionic surfactants.

Examples of the anionic surfactant include fatty acid salts such as sodium laurate (carbon number: 12), potassium laurate (carbon number: 12), potassium myristate (carbon number: 14), potassium palmitate (carbon number: 16), potassium stearate (carbon number: 18) and triethanolamine laurate (carbon number: 18); amino acid salts such as sodium cocoyl glutamate, sodium N-lauroyl-L-aspartate, sodium coconut fatty acid sarcosine, coconut fatty acid sarcosine triethanolamine, sodium lauroyl sarcosine and sodium lauroyl methylalanine; alkyl sulfate esters such as sodium lauryl sulfate and sodium stearyl sulfate; alkyl ether sulfates such as sodium laureth sulfate and other polyoxyethylene alkyl ether sulfates; phosphate ester salts such as sodium lauryl phosphate; sulfonates such as sodium dodecyl benzene sulfonate; and sulfosuccinate esters such as disodium laureth sulfosuccinate.

Examples of the cationic surfactant include alkyl type quaternary ammonium salts such as stearyltrimethylammonium chloride and behentrimonium chloride; benzalkonium type quaternary ammonium salts such as benzalkonium chloride and benzethonium chloride.

Examples of the ampholytic surfactant include alkylbetaines such as lauryl dimethylaminoacetate betaine and stearyl dimethylaminoacetate betaine; imidazoline type betaines such as disodium cocoamphodiacetate; betaine type ampholytic surfactants such as lauramidopropyl betaine (lauroylpropyl betaine) and cocamidopropyl betaine and other alkylamidopropyl betaines; and amino acid type ampholytic surfactants such as sodium beta-laurylaminopropionate.

Examples of the nonionic surfactant include sorbitan fatty acid esters such as sorbitan isostearate, sorbitan laurylate and sorbitan palmitate; polyoxyalkylene fatty acid esters such as polyethylene glycol monostearate ester and polyethylene glycol distearate ester; polyglycerin fatty acid esters such as decaglyceryl monostearate and decaglyceryl monolaurate; glycerin fatty acid esters such as glyceryl stearate, glyceryl isostearate, glyceryl oleate and glyceryl caprylate; polyoxyethylene glyceryl fatty acid esters such as polyethylene glycol-20 glyceryl isostearate, polyethylene glycol-60 glyceryl isostearate, PEG-120 methylglucose triisostearate, polyethylene glycol-5 glyceryl stearate and polyethylene glycol-7 glyceryl coconut oil fatty acid ester; polysorbates such as polysorbate 20, polysorbate 60 and polysorbate 80; and alkylalkanol type nonionic surfactants such as cocamidomethyl MEA.

The surfactant may be used either individually or in combination of two or more of such surfactants. The surfactant may be either commercially available or obtained by extraction from natural products or chemical synthesis.

The content of surfactant in the personal care composition is not particularly limited, and may be any amount normally employed in a personal care composition. For example, from the viewpoint of the cleansing property and the skin feel of the resulting personal care composition, relative to 100 parts by mass of low substituted hydroxypropyl cellulose, the content is preferably 10 parts by mass to 10,000 parts by mass, more preferably 100 parts by mass to 1,000 parts by mass, and still more preferably 200 parts by mass to 600 parts by mass.

### <Additives>

The personal care composition may further contain other components (i.e., additives) in addition to water and low substituted hydroxypropyl cellulose so as to have the desired properties. Examples of the additives include those normally employed in a personal care composition, such as solvents, thickening agents, antioxidants, pH adjusting agents, preservative agents, moisturizers, pearling agents, clay minerals, astringents, whitening agents, colorants, fragrances, beauty ingredients, cooling agents and powders, which are different from water or low substituted hydroxypropyl cellulose. Specific examples of the additives are listed below, but the additives are not limited to those listed below.

Examples of the solvent include polyhydric alcohols such as glycerin, diglycerin, butylene glycol, propylene glycol and dipropylene glycol; alcohols such as ethanol; hydrocarbon oils such as liquid paraffin and squalane; natural oils such as sunflower oil, macadamia nut oil, avocado oil, almond oil, olive oil, jojoba oil and coconut oil; ester oils such as 2-ethylhexyl isononanoate, isononyl isononanoate, isopropyl myristate, isopropyl palmitate, octyl palmitate, isopropyl isostearate, cetyl 2-ethylhexanoate, tri (caprylic/capric) glycerin and glycerin tri-2-ethylhexanoate; and silicone oils such as decamethylcyclopentasiloxane, octamethylcyclotetrasiloxane, aminopropyl dimethyl silicone, dimethyl silicone and diphenyl silicone.

Examples of the thickening agent include water-soluble polymers such as xanthane gum, guar gum, gellan gum, locust bean gum, carboxymethyl cellulose, hydroxyethyl cellulose, hydroxypropylmethyl cellulose, hydrophobized hydroxypropylmethyl cellulose, methylcellulose, cationized hydroxyethyl cellulose, carboxyvinyl polymer and polyvinyl alcohol.

Examples of the pH adjusting agent include citric acid, sodium citrate, gluconic acid, succinic acid, sodium hydroxide and potassium hydroxide.

Examples of the antioxidant include tocopherol, tocopherol acetate, butyl hydroxyanisole and dibutyl hydroxytoluene.

Examples of the preservative agent include methylparaben, ethylparaben, propylparaben, butylparaben and phenoxyethanol.

Examples of the moisturizer include hyaluronic acid, sodium hyaluronate, polyethylene glycol, mucopolysaccharide, urea, sorbitol, chondroitin sulfate, pyrrolidone carboxylic acid, sodium lactate and polyaspartic acid.

Examples of the pearlizing agent include ethylene glycol monostearate, ethylene glycol monobehenate, ethylene glycol distearate and ethylene glycol dibehenate.

Examples of the clay mineral include mica, sericite, talc, kaolin, montmorillonite, saponite, hectorite and smectite.

Examples of the astringent include zinc oxide, zinc paraphenol sulfonate, aluminum hydroxychloride, allantoin dihydroxyaluminum, peppermint extract, aloe extract, hamamelis extract, rosemary extract, lavender extract and eucalyptus extract.

Examples of the whitening agent include arbutin, α-arbutin, ascorbic acid; ascorbic acid fatty acid esters such as sodium ascorbate phosphate, magnesium ascorbate phosphate and tetraisopalmitate ascorbate; kojic acid, ellagic acid and tranexamic acid; and the derivatives thereof.

Examples of the fragrance include natural fragrances and synthetic fragrances.

Examples of the natural fragrance include rose oil, jasmine oil, lavender oil, ylang-ylang oil, peppermint oil, geranium oil, patchouli oil, sandalwood oil, cinnamon oil, lemon oil, orange oil and bergamot oil.

Examples of the synthetic fragrance include limonene, β-caryophyllene, cis-3-hexenol, linalool, farnesol, β-phenylethyl alcohol, 2,6-nonadienal, citral, α-hexyl cinnamaldehyde, iota-carvone, cyclopentadecanone, linalyl acetate, γ-undecalactone, aurantiol and I-menthol.

The personal care composition according to one embodiment of the present invention may contain one or more scrubbing agents other than low substituted hydroxypropyl cellulose as one type of additives, as long as the problems of the present invention can be solved. Specific examples of the scrubbing agent include gelatinized starch powder, cellulose powder, cellulose acetate powder, silica powder, polyhydroxyalkanoate particles and polylactic acid derivative particles. However, in view of the good skin feel, the personal care composition according to one embodiment of the present invention preferably contains only low substituted hydroxypropyl cellulose as a scrubbing agent.

The additives may be used either individually or in combination of two or more of such additives. The additives may be either commercially available or obtained by extraction from natural products or chemical synthesis.

The content of additives in the personal care composition is not particularly limited, and may be any amount normally employed in a personal care composition. For example, relative to 100 parts by mass of low substituted hydroxypropyl cellulose, the content is preferably from 0 parts by mass to 10,000 parts by mass, more preferably from 1 part by mass to 1,000 parts by mass, and still more preferably from 5 parts by mass to 500 parts by mass.

### <Personal care composition>

The personal care composition according to one embodiment of the present invention contains the above-mentioned low substituted hydroxypropyl cellulose and water thereby providing the technical features of low environmental impact, good skin feel and high cleansing property. So long as the personal care composition according to one embodiment of the present invention has such technical features, other properties of the composition are not particularly limited.

The personal care composition can be produced, for example, by mixing water and low substituted hydroxypropyl cellulose, and, if necessary, a surfactant and other additives. While the process of mixing the components for the personal care composition is not particularly limited, the process may be achieved by appropriately selecting the means and conditions of uniformly mixing the components.

The usage of the personal care composition is not particularly limited as long as the composition is applied for body care. The personal care composition may be in any form of formulations including formulations which remain on the body after application and formulations which are washed off the body after application.

While the body part of applying the personal care composition is not particularly limited, preferable examples of the body part include body parts such as skin and hair to be targeted by topical products and mucosal tissues such as eye and mouth. In addition, the personal care composition may be orally applied for the purpose of cleansing the gastrointestinal tract and other purposes.

The dosage form of the personal care composition is not particularly limited as long as the personal care composition is in a dosage form to demonstrate the good cleansing property and skin feel. Examples of the dosage form include liquid, cream, gel, lotion and foam. Among such dosage forms, the personal care composition is preferably in liquid form in order to demonstrate the excellent cleansing property and skin feel. The personal care composition may also be obtained by encapsulating the composition in the above dosage form in a capsule container or other materials.

The specific embodiments of the personal care composition include, but are not limited to, makeup removers (cleansing products), facial cleansing products, serums, toners, emulsions, shampoos, conditioners, hair treatments, hair styling products, shaving foam, face washes, hand washes, mouthwashes, eyewashes, skin toners, skin fresheners, soluble bath oils, solid soaps, sunscreens and body milks.

The personal care composition according to one embodiment of the present invention has the more excellent cleansing property and skin feel as compared to the personal care composition produced by replacing the above-mentioned low substituted hydroxypropyl cellulose with a low substituted hydroxypropyl cellulose having an average particle size (D₅₀) on a volume basis by dry laser diffraction of a value out of the range between 30 µm and 200 µm and an aspect ratio of a value out of the range equal to or more than 1.0 and less than 5.0 (hereinafter referred to as "comparative personal care composition").

The cleansing property and skin feel provided by the personal care composition according to one embodiment of the present invention can be evaluated by the cleansing property evaluation and sensory evaluation as described in Examples below.

The cleansing property according to one embodiment of the present invention, for example, may be an extent that the colored area ratio as measured by the cleansing property evaluation is preferably less than 4.9 %, more preferably equal to or less than 4.8 %, still more preferably equal to or less than 4.5 %, and even still more preferably equal to or less than 4.0 % or 3.5 %.

The skin feel of the personal care composition according to one embodiment of the present invention, for example, may be an extent that the average point of the sensory evaluation in view of the skin feel is preferably equal to or more than 2.0, and more preferably equal to or more than 2.5.

A preferred embodiment of the present invention is the personal care composition with which the colored area ratio as measured by the cleansing property evaluation is less than 4.9 %, and the average point of the sensory evaluation in view of the skin feel is equal to or more than 2.0. Another preferred embodiment of the present invention is the personal care composition with which the colored area ratio as measured by the cleansing property evaluation is equal to or less than 3.5 %, and the average point of the sensory evaluation in view of the skin feel is equal to or more than 2.5.

### <Other aspects of the invention>

Another aspect of the present invention relates to a method for cleansing a body, including the step of applying the personal care composition according to one embodiment of the present invention to the body to cleanse the body.

Another aspect of the invention is a method of cleansing a body part, including the step of applying the personal care composition according to one embodiment of the present invention to at least one body part selected from the group consisting of skin, hair and mucous membrane to cleanse the body part.

While the method for applying the personal care composition according to one embodiment of the present invention to the body or the body part is not particularly limited, examples of the method include putting drops of the personal care composition according to one embodiment of the present invention onto the body part followed by spreading or rubbing the personal care composition using fingers, hands or application tools so that the personal care composition can sufficiently get contact with the body part. For example, a certain amount of the personal care composition may be applied to eye or mouth followed by getting contact with the body part through voluntary movements by blinking or gargling.

### Examples

While the present invention will now be described in further detail with reference to Synthesis Examples, Examples and Comparative Examples, the present invention is not limited to these Synthesis Examples and Examples.

### <Preparation of low substituted hydroxypropyl cellulose>

[Synthesis Example 1: Preparation of L-HPC1] Wood-derived sheet pulp was soaked in 43 %(w/w) aqueous sodium hydroxide solution at 35 °C for 5 seconds and then pressed to remove the excess aqueous sodium hydroxide solution to obtain alkali cellulose containing 22.0 %(w/w) sodium hydroxide (in which the mass ratio of sodium hydroxide relative to anhydrous cellulose in alkali cellulose was 0.479).

The resulting alkali cellulose was shredded by a slitter cutter. The shredded alkali cellulose, which was 100 parts by mass as anhydrous cellulose amount, was fed into an internally stirred pressurized reactor, and the nitrogen gas was thoroughly filled into the vessel of the reactor. After the nitrogen replacement, 27 parts by mass of propylene oxide were added to the reactor, and the contents of the reactor were reacted at 50 °C for 3 hours to obtain a reaction product.

The reaction product, which was 100 parts by mass as anhydrous cellulose amount, was fed into a kneader including the mixed solution composed of 500 parts by mass of warm water at 45 °C and 14.4 parts by mass of acetic acid (20 % of the neutralization equivalent) and then dispersed in the mixed solution to obtain a reaction product dispersion solution. The reaction product dispersion solution in the kneader was mixed at a jacket temperature of 45 °C for 40 minutes (rotation speed: 43 rpm) to dissolve a portion of the reaction product. The resulting solution was completely neutralized by adding 57.5 parts by mass of acetic acid (80 % of the neutralization equivalent) thereto to precipitate a crude low substituted hydroxypropyl cellulose.

The precipitated crude low substituted hydroxypropyl cellulose was dispersed in 3,000 parts by mass of hot water at about 90 °C to obtain a crude low substituted hydroxypropyl cellulose dispersion solution. The crude low substituted hydroxypropyl cellulose dispersion solution was subjected to centrifugal separation treatment using a batch type centrifuge (rotation speed: 3,000 rpm) to wash and dehydrate the crude low substituted hydroxypropyl cellulose. After the centrifugation, the crude low substituted hydroxypropyl cellulose was dried at 80 °C for 18 hours using a shelf-stage dryer. The dried crude low substituted hydroxypropyl cellulose was pulverized using an impact mill ("Victory Mill VP-1", manufactured by Hosokawa Micron) followed by being sieved through a sieve with a mesh aperture of 75 µm to obtain low substituted hydroxypropyl cellulose as a pulverized product.

For the resulting low substituted hydroxypropyl cellulose, the content of hydroxypropoxy group, D₅₀ by dry method, D₅₀ by wet method and the aspect ratio were measured. The measurement results are shown in Table 1.

[Synthesis Example 2: Preparation of L-HPC2] The reaction product was obtained in the same manner as in Synthesis Example 1, except that the amount of propylene oxide supplied was 20 parts by mass.

The reaction product, which was 100 parts by mass as anhydrous cellulose amount, was fed into a kneader including the mixed solution composed of 500 parts by mass of warm water at 45 °C and 7.2 parts by mass of acetic acid (10 % of the neutralization equivalent) and then dispersed in the mixed solution to obtain a reaction product dispersion solution. The reaction product dispersion solution in the kneader was mixed at a jacket temperature of 45 °C for 40 minutes (rotation speed: 43 rpm) to dissolve a portion of the reaction product. The resulting solution was completely neutralized by adding 64.7 parts by mass of acetic acid (90 % of the neutralization equivalent) thereto to precipitate a crude low substituted hydroxypropyl cellulose.

The precipitated crude low substituted hydroxypropyl cellulose was used to obtain low substituted hydroxypropyl cellulose in the same manner as in Synthesis Example 1.

For the resulting low substituted hydroxypropyl cellulose, the content of hydroxypropoxy group, D₅₀ by dry method, D₅₀ by wet method and the aspect ratio were measured. The measurement results are shown in Table 1.

[Synthesis Example 3: Preparation of L-HPC3] Into an internally stirred pressurized reactor, wood-derived pulverized pulp, 100 parts by mass as anhydrous cellulose amount, was fed, and then 75 parts by mass of 35 %(w/w) aqueous sodium hydroxide solution was fed while stirring. The contents of the reactor were then mixed at a jacket temperature of 45 °C for 30 minutes to obtain alkali cellulose containing 15.0 %(w/w) sodium hydroxide (the mass ratio of sodium hydroxide relative to anhydrous cellulose in alkali cellulose was 0.281). After the nitrogen gas was thoroughly filled into the vessel of the reactor, 19 parts by mass of propylene oxide were added to the reactor, and the contents of the reactor were reacted at a jacket temperature of 60 °C for 2 hours while stirring in order to obtain a reaction product.

After 4,220 parts by mass (100 % of the neutralization equivalent) of 1 %(w/w) acetic acid was fed into the reactor, the contents of the reactor were stirred to neutralize the reaction product to precipitate a crude low substituted hydroxypropyl cellulose. The precipitated crude low substituted hydroxypropyl cellulose was subjected to centrifugal separation treatment (rotation speed: 3,000 rpm) using a batch type centrifuge to be washed and dehydrated, and then was dried in a shelf-stage dryer at 80 °C for 18 hours.

The dried crude low substituted hydroxypropyl cellulose was pulverized using a batch type planetary ball mill ("P-5", manufactured by FRITSCH) at 255 rpm for 60 minutes followed by being sieved through a sieve with a mesh aperture of 106 µm to obtain low substituted hydroxypropyl cellulose as a pulverized product.

For the resulting low substituted hydroxypropyl cellulose, the content of hydroxypropoxy group, D₅₀ by dry method, D₅₀ by wet method and the aspect ratio were measured. The measurement results are shown in Table 1.

[Synthesis Example 4: Preparation of L-HPC4] The reaction product was obtained in the same manner as in Synthesis Example 1, except that the amount of propylene oxide supplied was 16 parts by mass.

The reaction product, which was 100 parts by mass as anhydrous cellulose amount, was fed into a kneader including the mixed solution composed of 600 parts by mass of warm water at 45 °C and 1.4 parts by mass of acetic acid (2 % of the neutralization equivalent) and then dispersed in the mixed solution to obtain a reaction product dispersion solution. The reaction product dispersion solution in the kneader was mixed at a jacket temperature of 45 °C for 50 minutes (rotation speed: 43 rpm) to dissolve a portion of the reaction product (low substituted hydroxypropyl cellulose). The resulting solution was completely neutralized by adding 70.5 parts by mass of acetic acid (98 % of the neutralization equivalent) thereto to precipitate a crude low substituted hydroxypropyl cellulose.

The precipitated crude low substituted hydroxypropyl cellulose was used to obtain low substituted hydroxypropyl cellulose in the same manner as in Synthesis Example 1.

For the resulting low substituted hydroxypropyl cellulose, the content of hydroxypropoxy group, D₅₀ by dry method, D₅₀ by wet method and the aspect ratio were measured. The measurement results are shown in Table 1.

[Comparative Synthesis Example 1: Preparation of L-HPC5] Low substituted hydroxypropyl cellulose was obtained in the same manner as in Synthesis Example 1, except that it was sieved through a sieve with a mesh aperture of 38 µm.

For the resulting low substituted hydroxypropyl cellulose, the content of hydroxypropoxy group, D₅₀ by dry method, D₅₀ by wet method and the aspect ratio were measured. The measurement results are shown in Table 1.

[Comparative Synthesis Example 2: Preparation of L-HPC6] The reaction product was obtained in the same manner as in Synthesis Example 1, except that the amount of propylene oxide supplied was 30 parts by mass.

The reaction product, which was 100 parts by mass as anhydrous cellulose amount, was fed into a kneader including the mixed solution composed of 500 parts by mass of warm water at 45 °C and 25.1 parts by mass of acetic acid (35 % of the neutralization equivalent) and then dispersed in the mixed solution to obtain a reaction product dispersion solution. The reaction product dispersion solution in the kneader was mixed at a jacket temperature of 45 °C for 40 minutes (rotation speed: 43 rpm) to dissolve a portion of the reaction product. The resulting solution was completely neutralized by adding 46.7 parts by mass of acetic acid (65 % of the neutralization equivalent) thereto to precipitate a crude low substituted hydroxypropyl cellulose.

The precipitated crude low substituted hydroxypropyl cellulose was washed, dehydrated, dried and pulverized in the same manner as in Synthesis Example 1 followed by being sieved through a sieve with a mesh aperture of 100 µm to obtain low substituted hydroxypropyl cellulose.

For the resulting low substituted hydroxypropyl cellulose, the content of hydroxypropoxy group, D₅₀ by dry method, D₅₀ by wet method and the aspect ratio were measured. The measurement results are shown in Table 1.

### <Measurement of physical property of low substituted hydroxypropyl cellulose>

[Content of hydroxypropoxy group] The content of hydroxypropoxy group was measured according to the quantification method "Assay" of the section "low substituted hydroxypropyl cellulose" in Japanese Pharmacopoeia, 18th Edition. In short:
- Weigh accurately about 65 mg of Low Substituted Hydroxypropylcellulose, transfer to a reaction vial, add 0.06 to 0.10 g of adipic acid, 2.0 mL of the internal standard solution (a solution of n-octane in o-xylene (3 in 100)) and 2.0 mL of hydroiodic acid, immediately stopper the vial tightly, and weigh accurately. Using a magnetic stirrer equipped in the heating module, or using a shaker, mix for 60 minutes while heating so that the temperature of the vial content is 130±2°C. Allow the vial to cool, and again weigh accurately. If the mass loss is less than 26 mg and there is no evidence of a leak of the content, use the upper layer of the mixture as the sample solution. Separately, put 0.06 to 0.10 g of adipic acid, 2.0 mL of the internal standard solution and 2.0mL of hydroiodic acid in a reaction vial, immediately stopper the vial tightly, and weigh accurately. Add 15 to 22mL of isopropyl iodide for assay through the septum using a micro-syringe, and weigh accurately. Shake the reaction vial thoroughly, and use the upper layer of the content as the standard solution. Perform the test with 1 to 2 mL each of the sample solution and standard solution as directed under Gas Chromatography <2.02> according to the following conditions: fused silica column 0.53 mm in inside diameter and 30 m in length, coated with dimethylpolysiloxane for gas chromatography in 3 mm thickness; He 4.3mL/min; split ratio 1:40; and calculate the ratios, QT and QS, of the peak area of isopropyl iodide to that of the internal standard.
- Amount (%) of hydroxypropoxy group (C₃H₇O₂) = M_{S}/M_{T} × QT/QS × 44.17
- M_{S}: Amount (mg) of isopropyl iodide for assay taken M_{T}: Amount (mg) of Low Substituted Hydroxypropylcellulose taken, calculated on the dried basis

[D₅₀ by dry method] D₅₀ by dry method was determined in the use of a laser diffraction particle size analyzer ("Mastersizer 3000", manufactured by Malvern) by measuring a particle size corresponding to 50 % cumulative value of volume-based cumulative distribution curve under the conditions of 2 bar of dispersion pressure and 2 % to 10 % scattering intensity in accordance with the dry method based on Fraunhofer diffraction theory.

[D₅₀ by wet method] D₅₀ by wet method was determined in the use of a relative concentration laser diffraction particle size analyzer ("LS13 320", manufactured by Beckman Coulter) by measuring a particle size corresponding to 50 % cumulative value of volume-based cumulative distribution curve under the conditions of a pump speed of 43 % and a relative concentration of 8 % to 12 % in accordance with the wet method based on Fraunhofer diffraction theory.

[Aspect ratio] The aspect ratio was determined by photographing at measurable magnification randomly selected 50 particles using a scanning electron microscope ("JSM-6010 LA", manufactured by JEOL) and measuring the long diameter (L) and short diameter (D) of each particle. The aspect ratio (L/D) value was calculated from the obtained diameters, and the average value (average aspect ratio) was obtained from the calculated aspect ratio values (n = 50).

### <Production of personal care composition>

[Materials used] L-HPC1 to L-HPC6 (listed in Table 1) prepared in Synthetic Examples 1 to 4 and Comparative Synthetic Examples 1 to 2 were used for low substituted hydroxypropyl cellulose.

Pure water was used for water.

The following surfactants were used: sodium lauroylsarcosine ("Fillet L", manufactured by NOF), lauroylpropyl betaine ("Nissan Anon BDI-SF", manufactured by NOF), sodium lauroylmethylalanine ("Softilt AS-L", manufactured by NOF), cocamidomethyl MEA ("Aminon C-11S", manufactured by Kao) and PEG-120 methylglucose triisostearate/tocopherol/water mixture ("MACBIOBRIDE MG-T", manufactured by NOF).

Citric acid (reagent, manufactured by Kishida Chemical) was used for the pH adjusting agent.

HPMC (methoxy group content: 23.2 % by mass, hydroxypropoxy group content: 9.5 % by mass, viscosity at 20 °C of 2 % by mass aqueous solution: 85,000 mPa.s, manufactured by Shin-Etsu Chemical) was used for the viscosity adjusting agent (thickening agent).

[Example 1] Sodium lauroylsarcosine (30.0 g), lauroylpropyl betaine (20.0 g), sodium lauroylmethyl alanine (20.0 g) and PEG-120 methylglucose triisostearate/tocopherol/water mixture (1.5 g) were accurately weighed and added to a 200ml beaker. The beaker containing the surfactants was heated in a hot water bath heated to 80 °C until it reached a constant temperature.

Pure water (26.95 g) heated to 98 °C was accurately weighed and added to a 50 ml beaker. HPMC (0.75 g) was added to the hot water and well dispersed. The beaker was transferred into a thermostatic bath at 5 °C, and the dispersion in the beaker was gently stirred while cooling in order to obtain an HPMC aqueous solution in which HPMC was dissolved in the hot water.

To the 200 ml beaker containing the surfactants, the prepared HPMC aqueous solution was added and stirred such that a uniform solution was obtained. Citric acid (0.3 g), cocamidomethyl MEA (2.0 g) and the low substituted hydroxypropyl cellulose L-HPC1 (5.0g) obtained in Synthesis Example 1 were further added to the beaker, and stirred such that a uniform solution, liquid personal care composition, was obtained.

The resulting personal care composition was evaluated for the cleansing property (colored area ratio) and skin feel. The measurement results are shown in Table 2.

[Example 2] The personal care composition was obtained in the same manner as in Example 1, except that the low substituted hydroxypropyl cellulose L-HPC2 obtained in Synthesis Example 2 was used. The resulting personal care composition was evaluated for the cleansing property and skin feel. The measurement results are shown in Table 2.

[Example 3] The personal care composition was obtained in the same manner as in Example 1, except that the low substituted hydroxypropyl cellulose L-HPC3 obtained in Synthesis Example 3 was used. The resulting personal care composition was evaluated for the cleansing property and skin feel. The measurement results are shown in Table 2.

[Example 4] The personal care composition was obtained in the same manner as in Example 1, except that the low substituted hydroxypropyl cellulose L-HPC4 obtained in Synthesis Example 4 was used. The resulting personal care composition was evaluated for the cleansing property and skin feel. The measurement results are shown in Table 2.

[Comparative Example 1] The personal care composition was obtained in the same manner as in Example 1, except that the low substituted hydroxypropyl cellulose L-HPC5 obtained in Comparative Synthesis Example 1 was used. The resulting personal care composition was evaluated for the cleansing property and skin feel. The measurement results are shown in Table 2.

[Comparative Example 2] The personal care composition was obtained in the same manner as in Example 1, except that the low substituted hydroxypropyl cellulose L-HPC6 obtained in Comparative Synthesis Example 2 was used. The resulting personal care composition was evaluated for the cleansing property and skin feel. The measurement results are shown in Table 2.

### <Evaluation methods of personal care composition>

[Cleansing property evaluation (colored area ratio)] The cleansing property was evaluated based on the percentage of colored area (colored area ratio). In other words, the evaluation was that the smaller the colored area ratio was, the higher the cleansing property was. The colored area ratio was measured using the personal care compositions of Examples 1 to 4 and Comparative Examples 1 to 2, and the digital microscope "VHX-7000" (manufactured by Keyence) in the following manner, and the average value and standard deviation were calculated (n=5 to 7).

The artificial skin bioskin plate "#W" (manufactured by Beaulax) was sectioned into 4 cm x 6 cm compartments. For the compartment of the bioskin plate, 0.1 g of foundation ("media cream foundation PO-B1", manufacture by Kanebo) was applied and spread all over. The foundation-applied bioskin plate was dried at room temperature for 10 minutes and then blow-dried at room temperature.

Onto the center of the compartment of the bioskin plate, 0.1 g of the personal care composition was dripped, and extended and blended with fingers in a circular motion 20 times. The bioskin plate to which the personal care composition was applied was rinsed with a stream of tap water and blow-dried at room temperature.

The dried bioskin plate was observed with the digital microscope. From the resulting observation images, the white areas on the bioskin plate and the colored areas due to residual stains on the bioskin plate were binarized, and the colored area ratio (= [colored areas] / [total area of white and colored areas] × 100) was calculated.

[Sensory evaluation: skin feel] The skin feel was confirmed by applying the following sensory evaluation by five panelists who excelled in the evaluation of the feel of cosmetics such as personal care compositions, using the personal care compositions of Examples 1 to 4 and Comparative Examples 1 to 2.

On the inner arm of the panelist, 0.5 g of the personal care composition was placed. The panelist scored the feel of the composition when spread according to the following evaluation criteria. The average value and standard deviation were obtained from the panelists' scoring results.
[Evaluation criteria] 3: Felt smooth and did not cause skin irritation.
2: Felt rough but did not cause skin irritation.
1: Felt strong skin irritation.

### <Evaluation results of personal care composition>

The personal care compositions of Examples 1 to 4 had the significantly reduced colored area ratio indicating the cleansing property by containing the low substituted hydroxypropyl celluloses L-HPC1 to L-HPC4 having an average particle size (D₅₀) on a volume basis by dry laser diffraction of 30 µm or more and an aspect ratio less than 5.0. Furthermore, the personal care compositions of Examples 1 to 4 had the excellent skin feel but almost caused no rough skin feel and skin irritation.

In Examples 2 and 4, the low substituted hydroxypropyl cellulose employed having the lower HPO substitution and less swellability allowed the personal care composition to enhance the dirt-removed effect and demonstrate more excellent cleansing property.

More specifically, the personal care composition of Comparative Example 1 containing the low substituted hydroxypropyl cellulose having the content of hydroxypropoxy group of 11 % by mass, an average particle size (D₅₀) by dry method of 28 µm and an aspect ratio of 3.6 had no problem in the skin feel, but had the poor cleansing property. Furthermore, when the low substituted hydroxypropyl cellulose employed was changed to one having a higher content of hydroxypropoxy group, a larger D₅₀ by dry method and a larger aspect ratio, the resulting personal care composition of Comparative Example 2 had the improved skin feel, but had the further deteriorated cleansing property. These results indicate that a personal care composition containing low substituted hydroxypropyl cellulose tends to have a good skin feel, and that the use of low substituted hydroxypropyl cellulose having a higher content of hydroxypropoxy group, a larger D₅₀ by dry method and a larger aspect ratio allows this tendency to increase but rather causes the personal care composition to have the deteriorated cleansing property.

When the low substituted hydroxypropyl cellulose used was changed to one having a larger D₅₀ by dry method and a similar aspect ratio as compared to the personal care composition of Comparative Example 1, the resulting personal care composition of Example 1 was superior in both cleansing property and skin feel to the personal care composition of Comparative Example 1.

Furthermore, when the low substituted hydroxypropyl cellulose used was changed to one having a larger D₅₀ by dry method and a smaller aspect ratio as compared to the personal care composition of Comparative Example 1, the resulting personal care composition of Example 3 had the desirable skin feel and further had the excellent cleansing property.

From the above results, it was found that when compared to the personal care composition of Comparative Example 1, the personal care composition containing the low substituted hydroxypropyl cellulose having a larger D₅₀ by dry method and a similar or smaller aspect ratio had the excellent cleansing property and skin feel. It is presumed that the excellent cleansing property and skin feel of the personal care composition would be achieved due to the synergistic effect of the swellability and particle properties of the low substituted hydroxypropyl cellulose employed, rather than to the individual effect of them.

On the other hand, when the low substituted hydroxypropyl cellulose used was changed to one having a lower content of hydroxypropoxy group as compared to the personal care composition of Example 1, the resulting personal care composition of Example 2 had the more excellent cleansing property. Furthermore, when the low substituted hydroxypropyl cellulose used was changed to one having a further lower content of hydroxypropoxy group as compared to the personal care composition of Example 1, the resulting personal care composition of Example 4 had the significantly excellent cleansing property.

From the above results, it was found that when compared to the personal care composition of Example 1, the use of low substituted hydroxypropyl cellulose having a similar or lower content of hydroxypropoxy group allowed the personal care composition to have the more excellent cleansing property. This leads to a presumption that the lower content of hydroxypropoxy group allows the low substituted hydroxypropyl cellulose to have less swellability so that the resulting personal care composition can have the enhanced dirt-removed effect and the improved cleansing property.

**[Table 1]**

| Items | | Content of hydroxypropoxy group (% by mass) | D₅₀ by dry method (µm) | Aspect ratio | D₅₀ by wet method (µm) |
|---|---|---|---|---|---|
| Synthesis Example 1 | L-HPC 1 | 11 | 53 | 3.8 | 100.1 |
| Synthesis Example 2 | L-HPC 2 | 8 | 47 | 3.8 | 76.3 |
| Synthesis Example 3 | L-HPC 3 | 11 | 38 | 2.2 | 67.1 |
| Synthesis Example 4 | L-HPC 4 | 6 | 41 | 3.8 | 70.4 |
| Comparative Synthesis Example 1 | L-HPC 5 | 11 | 28 | 3.6 | 49.4 |
| Comparative Synthesis Example 2 | L-HPC 6 | 12 | 62 | 5.0 | 106.5 |

**[Table 2]**

| | Personal care compositions | | | | | | | | | Cleansing property | | Sensory evaluation | |
|---|---|---|---|---|---|---|---|---|---|---|---|---|---|
| | Scrubbing agents | Surfactants | | | | | pH adjusting agents | Thickening agents | Water | Colored area ratio (%) | | Skin feel (Scores) | |
| | (wt%) | (wt%) | (wt%) | (wt%) | (wt%) | (wt%) | (wt%) | (wt%) | (wt%) | Ave. | S.D. | Ave. | S.D. |
| Example 1 | L-HPC1 | *Surfactant 1 | *Surfactant 2 | *Surfactant 3 | *Surfactant 4 | *Surfactant 5 | Citric acid | HPMC | Pure water | 2.4 | 0.8 | 3.0 | 0 |
| | [5.0] | [6.0] | [5.6] | [6.0] | [2.0] | [1.5] | [0.3] | [0.75] | [77.85] | | | | |
| Example 2 | L-HPC2 | *Surfactant 1 | *Surfactant 2 | *Surfactant 3 | *Surfactant 4 | *Surfactant 5 | Citric acid | HPMC | Pure water | 1.6 | 1.1 | 2.2 | 0.4 |
| | [5.0] | [6.0] | [5.6] | [6.0] | [2.0] | [1.5] | [0.3] | [0.75] | [77.85] | | | | |
| Example 3 | L-HPC3 | *Surfactant 1 | *Surfactant 2 | *Surfactant 3 | *Surfactant 4 | *Surfactant 5 | Citric acid | HPMC | Pure water | 3.3 | 1.6 | 3.0 | 0 |
| | [5.0] | [6.0] | [5.6] | [6.0] | [2.0] | [1.5] | [0.3] | [0.75] | [77.85] | | | | |
| Example 4 | L-HPC4 | *Surfactant 1 | *Surfactant 2 | *Surfactant 3 | *Surfactant 4 | *Surfactant 5 | Citric acid | HPMC | Pure water | 0.9 | 1.0 | 2.2 | 0.4 |
| | [5.0] | [6.0] | [5.6] | [6.0] | [2.0] | [1.5] | [0.3] | [0.75] | [77.85] | | | | |
| Corrparative Example 1 | L-HPC5 | *Surfactant 1 | *Surfactant 2 | *Surfactant 3 | *Surfactant 4 | *Surfactant 5 | Citric acid | HPMC | Pure water | 4.9 | 1.7 | 2.2 | 0.4 |
| | [5.0] | [6.0] | [5.6] | [6.0] | [2.0] | [1.5] | [0.3] | [0.75] | [77.85] | | | | |
| Comparative Example 2 | L-HPC6 | *Surfactant 1 | *Surfactant 2 | *Surfactant 3 | *Surfactant 4 | *Surfactant 5 | Citric acid | HPMC | Pure water | 6.4 | 6.7 | 3.0 | 0 |
| | [5.0] | [6.0] | [5.6] | [6.0] | [2.0] | [1.5] | [0.3] | [0.75] | [77.85] | | | | |

| | | | | | | | | | | | | | |
|---|---|---|---|---|---|---|---|---|---|---|---|---|---|
| *Surfactant 1: sodium lauroylsarcosine *Surfactant 2: lauroylpropyl betaine *Surfactant 3: sodium lauroylmethylalanine *Surfactant 4: cocamidomethyl MEA *Surfactant 5: PEG-120 methylglucose triisostearate/tocopherol/water mixture | | | | | | | | | | | | | |

### Industrial Applicability

According to the present invention, a personal care composition having not only a low environmental impact and a good skin feel but also a high cleansing property can be produced. The personal care composition according to one embodiment of the present invention can be placed in wide distribution. The personal care composition according to one embodiment of the present invention can be produced and available on an industrial scale.

## Claims

1. A personal care composition comprising water and low substituted hydroxypropyl cellulose, wherein the low-substituted hydroxypropyl cellulose has an average particle size (D50) on a volume basis by dry laser diffraction of 30 µm to 200 µm and an aspect ratio equal to or more than 1.0 and less than 5.0, and wherein the low substituted hydroxypropyl cellulose has a content of hydroxypropoxy group of 4.0 % by mass to 11.0 % by mass.

2. The personal care composition according to claim 1, wherein a ratio of the mass of water relative to the mass of low substituted hydroxypropyl cellulose is equal to or more than 3.0; particularly wherein the ratio of the mass of water relative to the mass of low substituted hydroxypropyl cellulose ranges from 4.5 to 99.0, more particularly from 6.0 to 95.0, and even more preferably from 7.5 to 90.0.

3. The personal care composition according to any one of claims 1 to 2, wherein the content of low substituted hydroxypropyl cellulose, relative to the total mass of the personal care composition, is in the range of 1.0 % by mass to 10.0 %, particularly in the range of 3.0 % by mass to 8.0 %.

4. The personal care composition according to any one of claims 1 to 3, wherein the low substituted hydroxypropyl cellulose is in the swollen state, wherein the swollen state of the low substituted hydroxypropyl cellulose contained in the personal care composition means the state that the particle of low substituted hydroxypropyl cellulose has absorbed water and water has penetrated into the particles so that the particle size is larger than that in the dry state, in the personal care composition and has an average particle size (D50) on a volume basis by wet laser diffraction of 50 µm to 400 µm; particularly from 55 µm to 300 µm, more particularly 60 µm to 200 µm, and even more particularly 60 µm to 150 µm, 60 µm to 120 µm or 60 µm to 100 µm.

5. The personal care composition according to any one of claims 1 to 4, further comprising a surfactant.

6. The personal care composition according to claim 5, wherein the surfactant is selected from an anionic surfactant, a cationic surfactant, an ampholytic surfactants and / or a nonionic surfactant.

7. The personal care composition according to claim 6, wherein the surfactant comprises sodium lauroylsarcosine, lauroylpropyl betaine, sodium lauroylmethylalanine, cocamidomethyl MEA and/or PEG-120 methylglucose triisostearate/tocopherol.

8. The personal care composition of any one of claims 1 to 7, wherein the personal care composition is a personal care liquid product.

9. Use of a personal care composition of any one of claims 1 to 8 as a product for cleansing the human body.

10. The use according to claim 9, wherein the product is selected from a makeup remover, a facial cleansing product, a shampoo, a. hair conditioner, a hair treatment or hair styling product, a shaving foam, a face washing product, a hand washing product, a mouthwash, an eyewash, a skin toner, a skin freshener, a soluble bath oil, a solid soap, a sunscreen and a body milk.

## Patentansprüche

1. Eine Körperpflegezusammensetzung, umfassend Wasser und niedrigsubstituierte Hydroxypropylcellulose, wobei die niedrigsubstituierte Hydroxypropylcellulose eine durchschnittliche Partikelgröße (D50) auf Volumenbasis, bestimmt mittels Trockenlaserdiffraktion, von 30 µm bis 200 µm und ein Seitenverhältnis von mindestens 1,0 und weniger als 5,0 aufweist, und wobei die niedrigsubstituierte Hydroxypropylcellulose einen Gehalt an Hydroxypropoxygruppen von 4,0 Massenprozent bis 11,0 Massenprozent aufweist.

2. Die Körperpflegezusammensetzung nach Anspruch 1, wobei das Verhältnis der Masse an Wasser zur Masse an niedrigsubstituierter Hydroxypropylcellulose gleich oder größer als 3,0 ist; insbesondere wobei das Verhältnis der Masse an Wasser zur Masse an niedrigsubstituierter Hydroxypropylcellulose im Bereich von 4,5 bis 99,0, insbesondere von 6,0 bis 95,0 und noch bevorzugter von 7,5 bis 90,0 liegt.

3. Die Körperpflegezusammensetzung nach einem der Ansprüche 1 bis 2, wobei der Gehalt an niedrigsubstituierter Hydroxypropylcellulose, bezogen auf die Gesamtmasse der Körperpflegezusammensetzung, im Bereich von 1,0 Massenprozent bis 10,0 Massenprozent, insbesondere im Bereich von 3,0 Massenprozent bis 8,0 Massenprozent, liegt.

4. Die Körperpflegezusammensetzung nach einem der Ansprüche 1 bis 3, wobei sich die niedrigsubstituierte Hydroxypropylcellulose im aufgequollenen Zustand befindet, wobei der aufgequollene Zustand der in der Körperpflegezusammensetzung enthaltenen niedrigsubstituierten Hydroxypropylcellulose den Zustand bezeichnet, in dem die Partikel der niedrigsubstituierten Hydroxypropylcellulose Wasser aufgenommen haben und Wasser in die Partikel eingedrungen ist, sodass die Partikelgröße größer ist als im trockenen Zustand, in der Körperpflegezusammensetzung und eine durchschnittliche Partikelgröße (D50) auf Volumenbasis mittels Nasslaserdiffraktion von 50 µm bis 400 µm aufweist; insbesondere von 55 µm bis 300 µm, genauer von 60 µm bis 200 µm und noch genauer von 60 µm bis 150 µm, von 60 µm bis 120 µm oder von 60 µm bis 100 µm.

5. Die Körperpflegezusammensetzung nach einem der Ansprüche 1 bis 4, die ferner ein Tensid umfasst.

6. Die Körperpflegezusammensetzung nach Anspruch 5, wobei das Tensid aus einem anionischen Tensid, einem kationischen Tensid, einem ampholytischen Tensid und/oder einem nichtionischen Tensid ausgewählt ist.

7. Die Körperpflegezusammensetzung nach Anspruch 6, wobei das Tensid Natriumlauroylsarcosin, Lauroylpropylbetain, Natriumlauroylmethylalanin, Cocamidomethyl-MEA und/oder PEG-120-Methylglucose-Triisostearat/Tocopherol umfasst.

8. Die Körperpflegezusammensetzung nach einem der Ansprüche 1 bis 7, wobei die Körperpflegezusammensetzung ein flüssiges Körperpflegeprodukt ist.

9. Verwendung einer Körperpflegezusammensetzung nach einem der Ansprüche 1 bis 8 als Produkt zur Reinigung des menschlichen Körpers.

10. Die Verwendung nach Anspruch 9, wobei das Produkt ausgewählt ist aus einem Make-up-Entferner, einem Gesichtsreinigungsprodukt, einem Shampoo, einer Haarspülung, einem Haarpflege- oder Haarstylingprodukt, einem Rasierschaum, einem Gesichtswaschmittel, einem Handwaschmittel, einem Mundwasser, einer Augenspülung, einem Hauttonikum, einem Hauterfrischer, einem löslichen Badeöl, einer festen Seife, einem Sonnenschutzmittel und einer Körpermilch.

## Revendications

1. Composition de soins personnels comprenant de l'eau et une hydroxypropylcellulose faiblement substituée, dans laquelle l'hydroxypropylcellulose faiblement substituée a une taille particulaire moyenne (D50) sur une base volumique par diffraction laser sèche de 30 µm à 200 µm et un rapport d'aspect supérieur ou égal à 1,0 et inférieur à 5,0, et dans laquelle l'hydroxypropylcellulose faiblement substituée a une teneur en groupe hydroxypropoxy de 4,0 % en masse à 11,0 % en masse.

2. Composition de soins personnels selon la revendication 1, dans laquelle un rapport de la masse d'eau par rapport à la masse d'hydroxypropylcellulose faiblement substituée est supérieur ou égal à 3,0 ; en particulier dans laquelle le rapport de la masse d'eau par rapport à la masse d'hydroxypropylcellulose faiblement substituée va de 4,5 à 99,0, plus particulièrement de 6,0 à 95,0, et encore plus préférablement de 7,5 à 90,0.

3. Composition de soins personnels selon l'une quelconque des revendications 1 à 2, dans laquelle la teneur en hydroxypropylcellulose faiblement substituée, par rapport à la masse totale de la composition de soins personnels, est dans la plage de 1,0 % en masse à 10,0 %, en particulier dans la plage de 3,0 % en masse à 8,0 %.

4. Composition de soins personnels selon l'une quelconque des revendications 1 à 3, dans laquelle l'hydroxypropylcellulose faiblement substituée est dans l'état gonflé, dans lequel l'état gonflé de l'hydroxypropylcellulose faiblement substituée contenue dans la composition de soins personnels signifie l'état dans lequel la particule d'hydroxypropylcellulose faiblement substituée a absorbé de l'eau et de l'eau a pénétré dans les particules de sorte que la taille particulaire est supérieure à celle dans l'état sec, dans la composition de soins personnels et a une taille particulaire moyenne (D50) sur une base volumique par diffraction laser humide de 50 µm à 400 µm ; en particulier de 55 µm à 300 µm, plus particulièrement de 60 µm à 200 µm, et encore plus particulièrement de 60 µm à 150 µm, de 60 µm à 120 µm ou de 60 µm à 100 µm.

5. Composition de soins personnels selon l'une quelconque des revendications 1 à 4, comprenant en outre un tensioactif.

6. Composition de soins personnels selon la revendication 5, dans laquelle le tensioactif est sélectionné parmi un tensioactif anionique, un tensioactif cationique, un tensioactif ampholyte et/ou un tensioactif non ionique.

7. Composition de soins personnels selon la revendication 6, dans laquelle le tensioactif comprend de la lauroylsarcosine de sodium, de la lauroylpropyle bétaïne, de la lauroylméthylalanine de sodium, du cocamidométhyle MEA et/ou du triisostéarate de méthylglucose PEG-120/tocophérol.

8. Composition de soins personnels selon l'une quelconque des revendications 1 à 7, dans laquelle la composition de soins personnels est un produit liquide de soins personnels.

9. Utilisation d'une composition de soins personnels selon l'une quelconque des revendications 1 à 8 en tant que produit pour nettoyer le corps humain.

10. Utilisation selon la revendication 9, dans laquelle le produit est sélectionné parmi un démaquillant, un produit de nettoyage du visage, un shampooing, un après-shampooing, un traitement capillaire ou produit de coiffage, une mousse à raser, un produit de lavage du visage, un produit de lavage des mains, un bain de bouche, un collyre, un tonique, une lotion rafraîchissante, une huile de bain soluble, un savon solide, un écran solaire et un lait corporel.
